# EUROPÄISCHE PATENTSCHRIFT

(11) **EP 3 233 142 B1**
(45) Veröffentlichungstag und Bekanntmachung des Hinweises auf die Patenterteilung: **06.02.2019**
(21) Anmeldenummer: 15804531.0
(22) Anmeldetag: 04.12.2015
(51) Int. Cl.: A61L 2/20, B67C 7/00, B65B 55/10, B65B 3/00

(54) **VERFAHREN UND VORRICHTUNG ZUM STERILISIEREN VON BEHÄLTERN**
METHOD AND DEVICE FOR STERILIZING CONTAINERS
PROCÉDÉ ET DISPOSITIF DE STÉRILISATION DE RÉCIPIENTS

(30) Priorität: 16.12.2014 DE 102014118776
(43) Veröffentlichungstag der Anmeldung: 25.10.2017
(73) Patentinhaber: SIG Technology AG, 8212 Neuhausen (CH)
(72) Erfinder: WEILER, Christian, 47608 Geldern (DE); GEISSLER, Hanno, 47802 Krefeld (DE); MAINZ, Hans-Willi, 52525 Heinsberg (DE); BOONKAEW, Sittipong, Chonburi 20260 (TH)
(74) Vertreter: Cohausz & Florack
(86) Internationale Anmeldenummer: PCT/EP2015/078618
(87) Internationale Veröffentlichungsnummer: WO 2016/096472

(56) Entgegenhaltungen:
- EP-A1- 0 361 858
- US-A- 4 992 247

## Beschreibung

Die Erfindung betrifft ein Verfahren zur Sterilisation von Behältern für, insbesondere fließfähige, Lebensmittel bei dem eine Wasserstoffperoxidlösung in einem Verdampfer verdampft wird, bei dem in einer Sterilisationszone wenigstens ein Behälter mit dem dampfförmigen Wasserstoffperoxid beaufschlagt wird, bei dem der unverbrauchte Teil des dampfförmigen Wasserstoffperoxids wenigstens teilweise aus der Sterilisationszone abgezogen wird. Ferner betrifft die Erfindung eine Vorrichtung zur Sterilisation von Behältern für, insbesondere fließfähige, Lebensmittel, vorzugsweise zur Durchführung des genannten Verfahrens, mit einer Sterilisationszone zur Sterilisation wenigstens eines Behälters, einem Verdampfer zum Verdampfen einer Wasserstoffperoxidlösung, einer Sterilisiereinrichtung zum Beaufschlagen des wenigstens einen Behälters mit dampfförmigem Wasserstoffperoxid in der Sterilisationszone und einer Abzugseinrichtung zum wenigstens teilweisen Abziehen des unverbrauchten Teils des dampfförmigen Wasserstoffperoxids aus der Sterilisationszone.

Verfahren zum Sterilisieren von Behältern für Lebensmittel sind in unterschiedlichen Ausgestaltungen bekannt. Beispielsweise wird in der US 4 992 247 A ein Verfahren zum Sterilisieren von Behältern mit dampfförmigem Wasserstoffperoxid in einer Sterilisationszone beschrieben, bei dem unverbrauchtes dampfförmiges Wasserstoffperoxid aus der Sterilisationszoen abgezogen und erneut einem Verdampfer zugeführt wird. Zudem wird in der EP 0 361 858 A1 ein Verfahren zum Sterilisieren von Behältern mit flüssigen Wasserstoffperoxid beschrieben.

Dabei erfolgen die Sterilisation und das Befüllen der Behälter mit, vorzugsweise fließfähigen, Lebensmitteln häufig in einer aseptischen Umgebung von ein und derselben Vorrichtung, die daher meist als Füllmaschine bezeichnet wird.

Zum Befüllen von Behältern mit, insbesondere fließfähigen, Lebensmitteln im industriellen Maßstab werden sogenannte Füllmaschinen eingesetzt. Da die Lebensmittel nach dem Befüllen und Verschließen der Behälter lange haltbar sein sollen, ist eine möglichst keimfreie Abfüllung wünschenswert. Hierzu weisen die Füllmaschinen Sterilisationsräume bzw. Aseptikkammern auf, in denen die Behälter sterilisiert und anschließend unter möglichst sterilen Bedingungen gefüllt sowie verschlossen werden.

Als Lebensmittelbehälter werden dabei vielfach Packungen verwendet, die an ihrer Oberseite offen sind, um eine Öffnung zum Befüllen bereitzustellen. Die Packungen können beispielsweise Kartonverbundpackungen sein, die aus einem Laminat umfassend eine Kartonschicht und äußere Kunststoffschichten, insbesondere Polyethylen (PE), gebildet sind. Der Karton verleiht den Packungen eine ausreichende Stabilität, damit die Packungen einfach gehandhabt und beispielsweise gestapelt werden können. Die Kunststoffschichten schützen den Karton vor Feuchtigkeit und die Lebensmittel vor einer Aufnahme unerwünschter Stoffe aus der Packung. Zusätzlich können noch weitere Schichten, wie etwa eine Aluminiumschicht, vorgesehen sein, die eine Diffusion von Sauerstoff und anderen Gasen durch die Packung vermindert.

Die Packungen können, vorzugsweise in der Füllmaschine, aus einem Packungsvorläufer gefertigt werden. Als Packungsvorläufer können beispielsweise Packstoffzuschnitte verwendet werden, die bedarfsweise vorkonfektioniert sein können, und zwar beispielsweise durch Siegeln der Längskanten zu einem Packstoffrohling . Alternativ kann das für die Packungsvorläufer verwendete Packungsmaterial quasi unendlich von einer Rolle abgewickelt werden. Im Falle eines Packstoffzuschnitts, wird dieser an Biegelinien gefaltet, um zunächst einen Packungsmantel und einen Packungsboden zu bilden. Durch Siegeln sich überlappender Abschnitte des Packstoffs werden der Packungsmantel und der Packungsboden geschlossen. Der Kopf der Packung bleibt zunächst noch offen. Bedarfsweise kann auch zunächst der Packungskopf verschlossen und die Packung durch den, vorzugsweise nach oben weisenden, noch offenen Boden befüllt werden.

Anschließend werden die Packungen in eine Sterilisationszone der Füllmaschine eingeschleust. Dies erfolgt meist, indem die Packungen nacheinander an die Packungen aufnehmenden Zellen einer Transportkette übergeben werden. Die Transportkette sorgt dann dafür, dass die Packungen mit definierter Geschwindigkeit und in definiertem Abstand zueinander durch die Sterilisationszone der Füllmaschine transportiert werden.

In der Sterilisationszone werden die Packungen zunächst vorgewärmt. Dazu werden die Behälter mit heißer Sterilluft angeblasen. Anschließend werden die Behälter sterilisiert. Dazu wird eine wässrige Wasserstoffperoxidlösung in einem Verdampfer verdampft. Der Dampf aus Wasser und Wasserstoffperoxid wird dann in die vorgewärmten Packungen eingedüst, wobei die innere Oberfläche des gesamten Packungsbehälters und wenigstens der Kopfbereich der äußeren Oberfläche mit dem Wasserstoffperoxid beaufschlagt werden. Das Wasserstoffperoxid reagiert mit den vorhandenen Mikroorganismen und tötet diese ab. Dies erfolgt schneller und mit weniger Kondensation, wenn die Behälter vorgewärmt werden. Anschließend erfolgt eine Trocknung der sterilisierten Packungen mit Sterilluft, wonach die Packung in die Füll- und Siegelzone übergeben wird und dort letztlich mit einem Lebensmittel befüllt wird. Das Lebensmittel ist dabei vorzugsweise fließfähig. In einer Vielzahl von Fällen handelt es sich bei dem Lebensmittel um Getränke. Anschließend wird die befüllte Packung noch verschlossen, bevor die verschlossene Packung über die Transportkette aus dem Füll- und Siegelzone transportiert wird. Die Sterilisationszone ist in Teilbereichen nach unten offen und endet auf Höhe der nicht sterilen Transportkette oder darunter. Die Mischung aus Sterilluft, Dampf und restlichem Wasserstoffperoxid wird daher am unteren Ende der Sterilisationszone abgezogen und abgeleitet.

In der Füll- und Siegelzone bildet sich eine sogenannte Aseptikzone aus. Die Aseptikzone bezeichnet den tatsächlich aseptischen Bereich im oberen Abschnitt der Füll- und Siegelzone. Die Aseptikkammer umfasst die Sterilisationszone sowie die Füll- und Siegelzone. Die Aseptikkammer kann nach Art eines Gehäuses ausgebildet sein, wobei Öffnungen zum Zuführen und Abführen von Packungen vorgesehen sind. Zudem kann die Aseptikkammer am unteren Ende wenigstens eine Öffnung aufweisen, um die Atmosphäre aus der Sterilisationszone und/oder der Füll- und Siegelzone abzuziehen. Der Raum unterhalb der Aseptikkammer ist nicht aseptisch, was jedoch die aseptische Abfüllung der Packung nicht beeinträchtigt.

Das in der Sterilisationszone nicht umgesetzte Wasserstoffperoxid wird nicht wiederverwendet. Bislang ist die Wiederverwertung von Wasserstoffperoxid stets als technisch zu aufwendig und als nicht wirtschaftlich angesehen worden. Es wurde daher lediglich versucht, die eingesetzte Menge an Wasserstoffperoxid zu verringern. Das Wasserstoffperoxid wird daher je nach den jeweiligen Anforderungen mit anderen Prozessabgasen einer Abgasreinigung zugeführt. Der auf diese Weise entstehende Verlust an Wasserstoffperoxid ist mit der Zeit jedoch nicht unerheblich. Außerdem kann das Wasserstoffperoxid durch seine hohe Reaktivität und oxidative Wirkung die Anlagentechnik zur Abgasbehandlung und/oder zum Abtransport des Abgases angreifen, weshalb meist Materialien verwendet werden, die vom Wasserstoffperoxid nicht oder nur geringfügig angegriffen werden.

Der vorliegenden Erfindung liegt daher die Aufgabe zugrunde, das Verfahren und die Vorrichtung jeweils der eingangs genannten Art derart auszugestalten und weiterzubilden, dass ein insgesamt wirtschaftlicherer Betrieb möglich ist.

Diese Aufgabe ist durch ein Verfahren gemäß Anspruch 1 gelöst, bei dem das abgezogene dampfförmige Wasserstoffperoxid in einem Kondensator wenigstens teilweise kondensiert und das kondensierte Wasserstoffperoxid erneut dem Verdampfer zugeführt wird.

Die genannte Aufgabe ist ferner durch eine Vorrichtung gemäß dem Oberbegriff von Anspruch 1 dadurch gelöst, dass ein Kondensator zum wenigstens teilweisen Kondensieren des abgezogenen dampfförmigen Wasserstoffperoxids und eine Zuführeinheit zum Zuführen des kondensierten Wasserstoffperoxids zum Verdampfer vorgesehen sind.

Die Erfindung hat also erkannt, dass eine wirtschaftliche Wiederverwertung wenigstens von Teilen des überstochiometrisch eingesetzten Wasserstoffperoxids im Sinne einer Kreislaufführung möglich ist. Dies ist trotz des für eine Kondensation, insbesondere von wasserdampfhaltigen Gasen typischerweise hohen Energieeinsatzes der Fall. Zudem hat die Erfindung erkannt, dass die verringerte Reaktivität und Selbstzersetzung des Wasserstoffperoxids im Kondensat zur Steigerung der Ausbeute genutzt werden kann. Alternativ oder zusätzlich muss das Kondensat nicht unmittelbar wiederverwendet werden, sondern kann zunächst ohne einen nennenswerten Verlust an Wasserstoffperoxid einer Konditionierung zugeführt werden.

Ein weiterer Vorteil der Erfindung liegt darin, dass durch die Prozessparameter der Kondensation, insbesondere der Temperatur, entweder die Wasserstoffperoxidkonzentration gesteigert oder der Wasserstoffperoxidverlust über die Gasphase verringert werden kann. Je geringer der Wasserstoffperoxidverlust, desto höher ist die sogenannte Wasserstoffperoxidausbeute bzw. Rückgewinnungsrate. So können beispielsweise Wasserstoffperoxidkonzentrationen im Kondensat realisiert werden, die zwischen 10 Gew.-% und 70 Gew.-% betragen. Vorzugsweise beträgt die Wasserstoffkonzentration zwischen 20 Gew.-% und 60 Gew.-%, insbesondere zwischen 25 Gew.-% und 40 Gew.-%. Die Rückgewinnungsrate des Wasserstoffperoxids kann zwischen 10% und 70% des dem Kondensator zugeführten Wasserstoffperoxids betragen. Bevorzugt beträgt die Rückgewinnungsrate jedoch zwischen 20% und 60%, insbesondere zwischen 30% und 50%. Alternativ oder zusätzlich kann die Wasserstoffkonzentration nach der Kondensation durch Konzentrieren oder Verdünnen des Kondensats auf einen Wert zwischen 30 Gew.-% und 40 Gew.-%, insbesondere etwa 35 Gew.-%, eingestellt werden.

Um das Volumen des auf die Packung geblasenen Sterilisationsgases zu erhöhen, besteht dies vorzugweise aus einem Behandlungsmittel umfassend Wasserdampf und Wasserstoffperoxid sowie aus Anteilen an, insbesondere gefilterter Luft, vorzugsweise Druckluft. So wird ein gleichmäßiger Kontakt zwischen Behälter und Wasserstoffperoxid erreicht, ohne erhöhte Mengen an Wasserstoffperoxid einsetzen zu müssen.

Dabei kann die Wasserstoffperoxidlösung der Einfachheit halber alternativ oder zusätzlich in einem elektrisch beheizten Verdampfer verdampft werden. Dann ist es problemlos möglich, beheizte Kontaktflächen für das Wasserstoffperoxid auf einem hohen Temperaturniveau bereitzustellen. Die Kontaktflächen werden dabei vorzugsweise von Heizwiderständen bereitgestellt, die an eine Spannungsversorgung angeschlossen sind. Damit die Verdampfung des Wasserstoffperoxids in einem Verdampfer zweckmäßig erfolgen kann, kann die gefilterte Luft, insbesondere gefilterte Druckluft zunächst mit der Wasserstoffperoxidlösung gemischt werden, bevor die Mischung aus Wasserstoffperoxidlösung und Luft im Verdampfer verdampft wird. Dies kann beispielsweise in einer Art Düse erfolgen, in der die Sterilluft als Trägergas zum Applizieren auf die Behälter und/oder zum Verwirbeln sowie Vermischen des Wasserstoffperoxids genutzt werden kann.

Ein sterilisierter Behälter wenigstens im Wesentlichen ohne Mikroorganismen auf der inneren Oberfläche eignet sich in besonderem Maße zum Befüllen mit Lebensmitteln, die zum leichteren Befüllen wenigstens fließfähig, insbesondere flüssig sein sollten. Dabei ist kein Eintrag von Mikroorganismen in den Behälter zwischen dem Sterilisieren und Befüllen erwünscht. Vor diesem Hintergrund ist es bevorzugt, wenn der sterilisierte Behälter in derselben Vorrichtung auch befüllt wird. Letztlich ist es also besonders bevorzugt, wenn es sich bei der Vorrichtung um eine Füllmaschine handelt und/oder das Verfahren auch den Schritt des Befüllens des wenigstens einen sterilisierten Behälters umfasst.

Die Sterilisationszone umfasst wenigstens den Bereich, in dem der zu sterilisierende Behälter mittels einer Sterilisiereinrichtung mit dampfförmigem Wasserstoffperoxid beaufschlagt wird, um Mikroorganismen abzutöten. Bedarfsweise erfolgen in der Sterilisationszone aber noch weitere Verfahrensschritte, die mit dem Einbringen von weiteren Medien über entsprechende Zugänge zur Sterilisationszone einhergehen können. Zudem kann die Sterilisationszone zusammen mit einer Füllzone und/oder einer Siegelzone Teile einer Aseptikkammer sein. Zur Vermeidung einer Kontamination des abgefüllten Produkts umfasst die Aseptikkamer neben der Sterilisationszone eine Füll- und Siegelzone. Dabei wird der Transport der Behälter und die Durchführung vereinfacht, wenn die Sterilisationszone und die Füll- und Siegelzone aneinander angrenzen. Um die Gefahr eines Eintrags von Mikroorganismen von der Sterilisationszone in die Füll- und Siegelzone zu verringern, kann zwischen der Sterilisationszone einerseits und der Füll- und Siegelzone andererseits eine bauliche Engstelle vorgesehen sein, die bedarfsweise lediglich etwas breiter sein kann als die Behälter, die durch die Engstelle von der Sterilisationszone in die Füll- und Siegelzone transportiert werden sollen. Alternativ oder zusätzlich kann die Sterilisationszone von der Füll- und Siegelzone durch einen sogenannten Vorhang getrennt sein, der durch eine, insbesondere laminare, Strömung von Sterilluft gebildet wird. Die Sterilluft strömt dabei vorzugsweise von oben nach unten, um den Eintrag von Mikroorganismen in die Füll- und Siegelzone zu vermeiden. Bei der Sterilluft kann es sich der Einfachheit halber um gefilterte Luft handeln. Die Luft wird, etwa mittels Membranen, so fein gefiltert, dass die in der Ausgangsluft vorhandenen Mikroorganismen soweit abgetrennt werden, dass die verbleibende Luft als steril angesehen werden kann.

Nachfolgend werden der einfacheren Verständlichkeit halber und zur Vermeidung unnötiger Wiederholungen bevorzugte Ausgestaltungen des Verfahrens und der Vorrichtung gemeinsam beschrieben, ohne im Einzelnen jeweils zwischen dem Verfahren und der Vorrichtung zu unterscheiden. Für den Fachmann ergeben sich aber dennoch jeweils die hinsichtlich des Verfahrens und hinsichtlich der Vorrichtung bevorzugten Merkmale.

Bei einer ersten bevorzugten Ausgestaltung der Erfindung wird der Sterilisationszone der Vorrichtung Sterilluft zugeführt. Dies kann das Eindringen von Keimen und dergleichen in die Sterilisationszone, insbesondere durch das Ausbilden einer sterilen Strömung aus Luft, Wasserdampf und Wasserstoffperoxid durch die Sterilisationszone, und zwar insbesondere von oben nach unten, verhindern helfen. Alternativ oder zusätzlich dient die Sterilluft nach einer Erwärmung derselben vorzugsweise der Vorwärmung und/oder der Trocknung des wenigstens einen Behälters, bedarfsweise ebenfalls in der Sterilisationszone. Auf diese Weise können auch Synergien erreicht werden. Dabei kann die Vorwärmung des Behälters vor der Sterilisation des Behälters erfolgen. Die die Mikroorganismen abtötenden Reaktionen laufen bei erhöhter Temperatur schneller ab. Außerdem kondensiert weniger des Behandlungsmittels in Form einer Mischung aus Wasserdampf und Wasserstoffperoxid in und/oder an dem Behälter. Sterilluft kann alternativ oder zusätzlich dazu dienen, den sterilisierten Behälter zu trocken, bedarfsweise ebenfalls in der Sterilisationszone. Bei der Sterilisation gebildetes Kondensat wird dabei vor dem Befüllen des Behälters entfernt. Wenigstens ein Teil der der Sterilisationszone zugeführten Sterilluft wird zusammen mit dem noch dampfförmigen Wasserstoffperoxid aus der Sterilisationszone ab- und dem Kondensator zur Kondensation des Wasserstoffperoxids zugeführt. Es kann aber auch alternativ oder zusätzlich zu einem etwaigen Schritt des Vorwärmens und/oder Trocknens des wenigstens einen Behälters bevorzugt sein, Sterilluft in die Sterilisationszone einzublasen. Dann kann infolge der Gasströmung der Sterilluft ein Eindringen von Fremdstoffen von außen in die Sterilisationszone verhindert werden.

Das Wasserstoffperoxid wird zur einfachen Verfahrensführung und zur Kostensenkung als wässrige Lösung dem Verdampfer zugeführt. Mithin wird dann der wenigstens eine Behälter mit dampfförmigem Wasserstoffperoxid und Wasserdampf, sowie bedarfsweise zusätzlich mit Sterilluft zum Verdünnen des Sterilgases, beaufschlagt. Dies bedeutet insbesondere, dass das dampfförmige Wasserstoffperoxid sowohl in den Behälter eingeleitet wird als auch mit der Außenseite des Behälters, wenigstens im Bereich der Öffnung, in Kontakt kommt. Die Außenseiten des Behälters können auch sterilisiert werden, da die Öffnung nach dem Befüllen des Behälters noch verschlossen werden muss. Ansonsten könnten dabei Mikroorganismen in den Behälter eindringen. Die Wasserstoffperoxidlösung kann dabei der Einfachheit halber aus einem Vorlagebehälter dem Verdampfer zugeführt werden. Der unverbrauchte Teil des Wasserstoffperoxids, also der Teil des Wasserstoffperoxids, der nicht bei der Sterilisation umgesetzt wurde, wird bevorzugt mit dem nicht kondensierten Wasserdampf und, bedarfsweise der Sterilluft, aus der Sterilisationszone abgezogen. Das entsprechende Gasgemisch wird anschließend dem Kondensator zugeführt, um das Wasserstoffperoxid und den Wasserdampf jeweils teilweise zu kondensieren. Im Kondensator wird das Gasgemisch dabei vorzugsweise auf eine Temperatur zwischen 35°C und 95°C abgekühlt. Die Temperatur kann je nach der gewünschten Wasserstoffperoxidkonzentration im Kondensat und/oder Rückgewinnungsrate des Wasserstoffperoxids gewählt werden. Mit steigender Temperatur wird weniger Wasserstoffperoxid zurückgewonnen aber eine höhere Wasserstoffperoxidkonzentration im Kondensat erzielt.

Bevorzugt weist die Sterilisationszone ein unteres Ende oder einen Boden auf, so dass das Wasserstoffperoxid bodenseitig abgezogen werden kann. Da das Wasserstoffperoxid zunächst der Einfachheit halber vorzugsweise von oben in den geöffneten Behälter geleitet wird, kann das dampfförmige Wasserstoffperoxid leicht von unten aus der Sterilisationszone abgezogen werden, ohne die Sterilisation zu beeinträchtigen. Wenn eine Transporteinrichtung, etwa eine Transportkette zum Transport des wenigstens einen Behälters durch die Vorrichtung, insbesondere die Füllmaschine, und/oder durch die Sterilisationszone vorgesehen ist, kann diese schwerlich steril gehalten werden. Wird das Wasserstoffperoxid unterhalb dieser Transporteinrichtung abgezogen, wird zudem eine Kontaminierung der Aseptikzone durch die Transporteinrichtung vermieden.

Die Wirtschaftlichkeit des Verfahrens kann gesteigert werden, wenn der Kondensator derart betrieben wird, dass das Kondensat eine höhere Wasserstoffperoxidkonzentration als die Wasserstoffperoxidlösung vor dem Verdampfer aufweist. Um dies sicherzustellen, kann eine bestimmte Kondensatortemperatur eingestellt werden oder es kann eine Steuereinheit vorgesehen sein, die die Kondensatortemperatur anhand der Wasserstoffperoxidkonzentration des Kondensats regelt. Als alternative oder zusätzliche Stellgröße zur Regelung der Wasserstoffperoxidkonzentration des Kondensats kommt zudem der Massenstrom der der Sterilisationszone zugeführten Sterilluft in Frage. Es kann aber auch zweckmäßig sein, wenn die Wasserstoffperoxidkonzentration des Kondensats geringer ist als die Wasserstoffperoxidlösung vor dem Verdampfer, etwa um die benötigte Menge an Wasserstoffperoxid zu reduzieren. Dann kann es jedoch erforderlich sein, ein Teil des Wassers aus dem Kondensat abzuscheiden und so das Wasserstoffperoxid aufzukonzentrieren. Dies kann durch wenigstens einen Separator zur Durchführung einer Umkehrosmose, ein Molekularsieb, einen Verdampfer oder andere Separatoren erfolgen. Ganz grundsätzlich ist es aus wirtschaftlichen Gesichtspunkten bevorzugt, wenn die Wasserstoffperoxidkonzentration zwischen 10 Gew.-% und 70 Gew.-%, beträgt. Dabei kann es für die effektive weitere Nutzung des Wasserstoffperoxids bevorzugt sein, wenn die Wasserstoffperoxidkonzentration wenigstens 15 Gew.-%, wenigstens 20 Gew.-% oder wenigstens 25 Gew.-% beträgt. Alternativ oder zusätzlich kann es etwa aus energetischen oder verfahrenstechnischen Gründen bevorzugt oder ausreichend sein, wenn die Wasserstoffperoxidkonzentration nicht mehr als 30 Gew.-%, 40 Gew.-% oder 50 Gew.-% beträgt.

Um eine geeignete Wasserstoffperoxidkonzentration für die Verdampfung und die Sterilisation bereitzustellen, kann das im Kondensator anfallende Kondensat auf eine vorgegebene Wasserstoffperoxidkonzentration zwischen 25 Gew.-% und 50 Gew.-%, weiter vorzugsweise zwischen 30 Gew.-% und 40 Gew.-%, insbesondere 33 Gew.-% und 37 Gew.-%, eingestellt werden. Dies lässt sich besonders einfach und kostengünstig bewerkstelligen, wenn die Wasserstoffperoxidkonzentration durch Zugabe von Wasser rückverdünnt, durch Zugabe von Wasserstoffperoxidlösung angehoben und/oder durch Abscheiden von Wasser gesteigert wird. Zur Verringerung des regelungstechnischen Aufwands erfolgt das Einstellen der Wasserstoffperoxidkonzentration vorzugsweise batchweise. Grundsätzlich führt die Sterilisation des wenigstens einen Behälters zu einem Verbrauch von Wasserstoffperoxid. Das verbrauchte Wasserstoffperoxid wird also vorzugsweise ersetzt, wobei das Wasserstoffperoxid der Einfachheit halber etwa mit der Konzentration zugeführt wird, die auch dem Verdampfer zugeführt wird. Das Zuführen von Wasserstoffperoxid macht dann keine oder allenfalls eine geringe Anpassung der Konzentration erforderlich. Auch hinsichtlich der Zuführung von Wasserstoffperoxid ist ein batchweises Vorgehen zur Verminderung des regelungstechnischen Aufwands vorteilhaft. Zur Einstellung der Konzentration an Wasserstoffperoxid und dem Auffüllen der Menge des Wasserstoffperoxids kann eine Konditionierungseinrichtung vorgesehen sein. Die Konditionierung kann dann batchweise in einem Vorlagebehälter erfolgen, wodurch der regelungstechnische Aufwand erheblich gesenkt werden kann. Dabei ist es auch bevorzugt, das insbesondere demineralisierte Wasser und die Wasserstoffperoxidlösung aus einem Vorlagebehälter dem Vorlagebehälter zuzuführen. Zur Abscheidung von Wasser aus dem Kondensat ist wirtschaftlich und energetisch die Verwendung von Molekularsieben besonders bevorzugt. Diese können beispielsweise aus Zeolithe und/oder Kohlenstoff gebildet sein und eine große innere Oberfläche, beispielsweise größer 500 m²/g, aufweisen. Die Porendurchmesser sind recht einheitlich sowie ähnlich der Größe der abzuscheidenden Wassermoleküle. Die mit Wasser beladenen Molekularsiebe können durch Erhitzen regeneriert werden, wobei das Wasser wieder ausgetrieben wird.

Das im Kondensator anfallende Kondensat kann mit Fremdstoffen, wie Staub, Bestandteilen des Behälters etc., angereichert sein. Daher ist es zweckmäßig, das Kondensat zu filtrieren. Als Filter kann ein Filter aus Polyethylen (PE) oder Polypropylen (PP) verwendet werden, da diese Materialien vom Wasserstoffperoxid nur geringfügig angegriffen werden und keinen katalytischen Einfluss auf die Zersetzung des Wasserstoffperoxid haben. Dies ist umso mehr der Fall, wenn als Filter ein gesintertes Polyethylen verwendet wird, das bevorzugt eine Porengröße zwischen 10 µm und 200 µm, insbesondere zwischen 30 µm und 50 µm oder zwischen 90 µm und 110 µm, aufweist. Wenn das Filtrat nach unten ablaufen kann, fällt der Filter trocken, so dass das Wasserstoffperoxid nicht mit den Filterrückständen reagiert. Ein weiterer Vorteil eines gesinterten Filters aus Polyethylen besteht in der kostengünstigen Fertigung und der einfachen Montage. Alternativ oder zusätzlich kann es zweckmäßig sein, das Kondensat, insbesondere nach der Filtration, einem Ionentauscher, vorzugsweise Kationentauscher zuzuführen. Durch den Ionentausch im Ionentauscher können beispielsweise Salzablagerungen verringert oder gar vermieden werden. Bei der Verwendung eines Kationentauschers verbleiben Säuren im Kondensat die zur Stabilisierung des Wasserstoffperoxids beitragen. Um den apparativen Aufwand gering zu halten, erfolgt das Filtrieren und/oder der Ionentausch vorzugsweise vor dem Einstellen der Wasserstoffperoxidkonzentration der dem Verdampfer zuzuführenden Lösung. Unabhängig davon kann die Filtereinrichtung Bestandteil der Konditionierungseinrichtung sein, um die Aufbereitung verfahrensmäßig und in kompakter Weise zusammenzuführen.

Für das Befüllen des Behälters ist es bevorzugt, wenn der wenigstens eine Behälter in der Sterilisationszone vor dem Beaufschlagen mit dampfförmigem Wasserstoffperoxid mit heißer Sterilluft vorgewärmt wird. Dann entsteht beim Sterilisieren weniger Kondensat und das Wasserstoffperoxid kühlt nicht so weit ab, so dass hohe Reaktionsgeschwindigkeiten erzielt werden. Alternativ oder zusätzlich kann der mittels dampfförmigem Wasserstoffperoxid sterilisierte Behälter mit Sterilluft getrocknet werden. So wird Kondensat vor dem Befüllen ausgetrieben und vermieden, dass im Behälter verbleibendes Wasserstoffperoxid das abzufüllende Lebensmittel oxidiert und/oder kontaminiert.

Unabhängig davon wird der Behälter nach dem Beaufschlagen mit dem dampfförmigen Wasserstoffperoxid und der dabei erfolgenden Sterilisation vorzugsweise mit einem Produkt in Form eines Lebensmittels befüllt. Das Verfahren bietet sich dabei insbesondere für fließfähige Produkte an, die wenigstens pastös oder bedarfsweise flüssig sein sowie bedarfsweise zusätzlich stückige Anteile enthalten können. Ganz besonders ist das beschriebene Verfahren zum Abfüllen von Getränken geeignet, bei denen aufgrund der abzufüllenden Mengen möglichst geringe Taktzeiten von besonderer wirtschaftlicher Bedeutung sind.

Das Befüllen von Behältern in kurzer Zeit kann begünstigt werden, wenn mehrere Behälter hintereinander mit Hilfe einer Transporteinrichtung durch die Aseptikkammer bzw. die Sterilisationszone und/oder die Füll- und Siegelzone transportiert werden. So lassen sich kurze Taktzeiten erreichen und Ausschuss vermeiden. Zum sicheren und definierten Transport der Behälter kann die Transporteinrichtung Zellen zur Aufnahme einzelner Behälter aufweisen. Dabei ist es alternativ oder zusätzlich konstruktiv besonders einfach, wenn die Transporteinrichtung als Transportkette ausgebildet ist.

Ganz grundsätzlich ist das zuvor beschriebene Verfahren besonders wirtschaftlich einzusetzen, wenn als Behälter eine Packung verwendet wird. Die Packung ist zudem vorzugsweise oben offen, um das Sterilisieren und Befüllen zu vereinfachen. Dabei kommt als Packung insbesondere eine Kartonverbundpackung in Frage. Dabei kann auch vorgesehen sein, dass vor dem Befüllen der Boden noch nicht verschlossen ist und nach oben weist. Dann ist vorzugsweise der Kopf der Packung bereits verschlossen, so dass die Packung durch den Boden gefüllt werden kann.

Wenn die Vorrichtung mit einer Filtereinrichtung versehen wird, kann die Filtereinrichtung enthaltende Fremdstoffe aus dem kondensierten Wasserstoffperoxid abscheiden, so dass eine Anreicherung entsprechender Fremdstoffe vermieden wird. Die Filtereinrichtung weist dazu insbesondere einen, bedarfsweise gesinterten, Filter aus Polyethylen und/oder Polypropylen auf, wie er zuvor bereits beschrieben worden ist. Die Fremdstoffe können dabei insbesondere Staub oder Abrieb der zu sterilisierenden Behälter sein.

Um die Konzentration der dem Verdampfer zuzuführenden Wasserstoffperoxidlösung einstellen zu können, so dass die Wasserstoffperoxidkonzentration der zum Sterilisieren verwendeten Lösung trotz der Kreislaufführung eines Teils des Wasserstoffperoxids in etwa konstant gehalten werden kann, weist die Vorrichtung vorzugsweise eine Konditionierungseinrichtung oder Einstelleinrichtung zum Einstellen der gewünschten Wasserstoffperoxidkonzentration im Kondensat auf. Die Konditionierungseinrichtung oder Einstelleinrichtung kann eine Verdünnungseinrichtung zum Verdünnen der Wasserstoffperoxidkonzentration umfassen, und zwar insbesondere durch Zugabe von Wasser. Die Verdünnungseinrichtung kann dazu an eine Wasserleitung angeschlossen sein oder einen Vorlagebehälter mit Wasser aufweisen. Es kann alternativ oder zusätzlich auch eine Konzentrierungseinrichtung vorgesehen sein, welche dem Anheben der Wasserstoffperoxidkonzentration dient. Dazu kann beispielsweise Wasserstoffperoxid zugegeben oder Wasser aus dem Kondensat abgetrennt werden. Zur Abtrennung von Wasser wird vorzugsweise ein Molekularsieb verwendet. Das Wasser kann grundsätzlich aber auch auf andere Weise abgeschieden werden. Das Einstellen der Wasserstoffperoxidkonzentration erfolgt vornehmlich diskontinuierlich also batchweise. Dies vereinfacht den regelungstechnischen Aufwand. Es ist aber dennoch eine Regelungseinrichtung zur Überwachung der Konzentrationseinstellung zweckmäßig. Ebenso kann eine kontinuierliche Einstellung der Wasserstoffperoxidkonzentration zweckmäßig sein. Dann muss weniger Wasserstoffperoxidlösung gehandhabt werden. Zudem ist weniger Bauraum für die Apparatetechnik erforderlich.

Alternativ oder zusätzlich kann die Vorrichtung eine Versorgungseinrichtung zum Ersetzen von nicht kondensiertem Wasserstoffperoxid aufweisen. Auf diese Weise kann einfach der Verlust an Wasserstoffperoxid, insbesondere infolge der Abtötung der Mikroorganismen und des den Kondensator über die Gasphase verlassenden Wasserstoffperoxidanteils, ausgeglichen werden. Die Versorgungseinrichtung umfasst daher vorzugsweise einen Vorlagebehälter mit einer Wasserstoffperoxidlösung, deren Konzentration in etwa der Konzentration der dem Verdampfer zuzuführenden Wasserstoffperoxidlösung entsprechen kann.

Zur Vereinfachung des Prozesses kann es bevorzugt sein, wenn die Filtereinrichtung, die Verdünnungseinrichtung und/oder die Versorgungseinrichtung in einer Konditionierungseinrichtung zusammengeführt werden. Die Konditionierungseinrichtung kann für das batchweise Konditionieren der Wasserstoffperoxidlösung wenigstens einen Vorlagebehälter umfassen. Aus dem wenigstens einen Vorlagebehälter kann dann nach erfolgter Konditionierung der Wasserstoffperoxidlösung der Vorlagebehälter der Wasserstoffperoxidlösung für den Verdampfer gespeist werden.

Das Befüllen der Behälter mit Lebensmitteln kann sicherer und schneller durchgeführt werden, wenn die Vorrichtung, insbesondere die Füllmaschine, eine Vorwärmeinrichtung zum Vorwärmen des wenigstens einen Behälters mit heißer Sterilluft, insbesondere in der Sterilisationszone, aufweist. Dann entsteht beim Sterilisieren weniger Kondensat und es kann eine hohe Reaktionskinetik sichergestellt werden, die eine zuverlässige Oxidation der Mikroorganismen gewährleistet. Alternativ oder zusätzlich kann eine Trocknungseinrichtung zur Trocknung des sterilisierten Behälters mit Sterilluft, insbesondere in der Sterilisationszone, vorgesehen sein. Kondensiertes Wasserstoffperoxid wird dann entfernt, das sonst anstelle des abzufüllenden Lebensmittels im Behälter verbleiben könnte. Zudem würde das Wasserstoffperoxid das abzufüllende Lebensmittel oxidieren, was unerwünscht sein kann. Zum sterilen Befüllen des sterilisierten Behälters bietet sich eine Fülleinrichtung an, die zum Füllen des sterilisierten Behälters mit Lebensmittel in der Füll- und Siegelzone ausgebildet ist. Damit auch nach dem Befüllen keine Kontamination des abgefüllten Lebensmittels stattfindet, kann eine Verschließeinrichtung zum Verschließen des gefüllten Behälters, insbesondere in der Füll- und Siegelzone, vorgesehen sein. Der Behälter wird dann erst nach dem Verschließen aus der Füll- und Siegelzone transportiert.

Zum Transport der Behälter weist die Vorrichtung, insbesondere die Füllmaschine, ganz grundsätzlich eine Transporteinrichtung auf, die insbesondere mehrere Behälter nacheinander durch die Sterilisationszone und/oder die Füll- und Siegelzone bzw. die Aseptikkammer transportieren kann. Um einen definierten Transport der Behälter in einem definierten räumlichen und zeitlichen Abstand sicherstellen zu können, um Ausschuss zu vermeiden, weist die Transporteinrichtung vorzugsweise Zellen auf, in die einzelne Behälter eingeführt und/oder gehalten werden können. Die Zellen sind dabei vorzugsweise so ausgebildet, dass deren Aufnahme mit äußeren Abmessungen der Behälter korrespondieren, was die Aufnahme der Behälter vereinfacht. Alternativ oder zusätzlich kann die Transporteinrichtung als Transportkette ausgebildet sein. Diese lässt sich einfach im Kreis führen, um einen regelmäßigen Vorschub und Nachschub der Behälter sicherstellen zu können.

Ganz grundsätzlich ist es besonders wirtschaftlich, wenn als Behälter eine Packung verwendet wird, die zum einfachen Sterilisieren und/oder Befüllen oben offen sein kann. Dabei kommt als Packung insbesondere eine Kartonverbundpackung in Frage, wie sie eingangs exemplarisch bereits beschrieben und aus dem Stand der Technik bekannt ist.

Um eine Sterilisation von Behältern in der beschriebenen Weise bei hoher Betriebssicherheit sicherstellen zu können, kann wenigstens eine Steuereinrichtung vorgesehen sein, welche die Steuerung und/oder Regelung einzelner Teilprozesse und/oder des Gesamtprozesses übernimmt.

Nachfolgend wird die Erfindung anhand einer lediglich Ausführungsbeispiele darstellenden Zeichnung näher erläutert. In der Zeichnung zeigt
- Fig. 1: ein Detail einer erfindungsgemäßen Vorrichtung in einer schematischen Darstellung und
- Fig. 2: ein erfindungsgemäßes Verfahren in einer schematischen Darstellung.

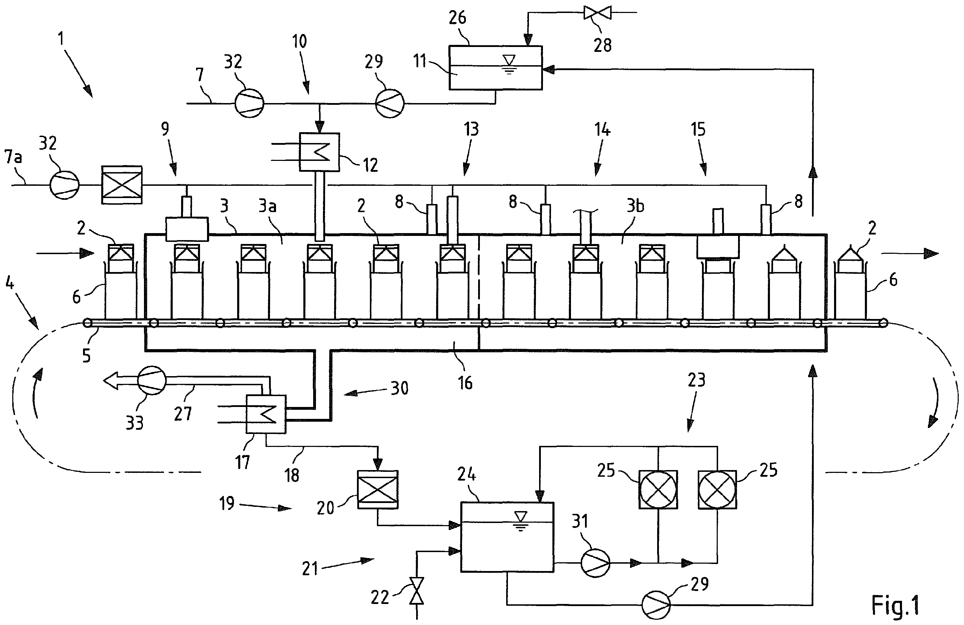

In der Fig. 1 ist ein Detail einer Vorrichtung 1 zum Sterilisieren von Behältern 2 in Form einer Füllmaschine schematisch dargestellt. Die dargestellte Vorrichtung 1 weist eine Aseptikkammer 3 auf, die eine Sterilisationszone 3a und eine Füll- und Siegelzone 3b umfasst, durch die eine Reihe von Behältern 2 in Form von Packungen wenigstens teilweise nacheinander hindurch transportiert werden. Die Transportrichtung der Behälter 2 weist dabei, wie durch die Pfeile symbolisiert wird, von links nach rechts, wobei der Transport der Behälter 2 jedoch nicht geradlinig erfolgen muss, sondern auch in einem Bogen oder gar im Kreis erfolgen kann. Die Behälter 2, in Form von Packungen, bei denen es sich insbesondere um Kartonverbundverpackungen mit wenigstens einer Kartonschicht, einer Aluminiumschicht und äußeren Schichten aus einem thermoplastischen Kunststoff, insbesondere Polyethylen (PE) handelt, werden mit Hilfe einer Transporteinrichtung 4 umfassend eine Transportkette 5 durch die Aseptikkammer 3 transportiert. Dazu werden die Behälter 2 in Form von Packungen vorzugsweise zunächst jeweils aus einem Packungszuschnitt durch Falten und teilweises Verschweißen bzw. Siegeln des Packungsmaterials gebildet. Dabei wird der Packungsboden gebildet und verschlossen. Der Kopf des Behälters bleibt offen, um den Behälter befüllen zu können. Es könnte aber auch der Kopf des Behälters geschlossen und der Behälter durch den noch unverschlossenen Boden befüllt werden.

Der Behälter 2 ist vorzugsmäßig quaderförmig ausgebildet und wird nach dem Formen an die Transporteinrichtung 4 in Form einer Transportkette 5 übergeben. Grundsätzlich können die Behälter aber auch eine andere Form aufweisen. Beispielsweise sind auch sphärische oder pyramidenförmige Behälter möglich. Dazu weist die Transporteinrichtung 4 Zellen 6 auf, in die die Behälter 2, die in der dargestellten Ausführungsform im unverschlossenen Zustand auch als Packungsrohlinge bezeichnet werden können, eingeführt werden. Die Behälter 2 bzw. Packungsrohlinge werden dann vorzugsweise formschlüssig in den Zellen 6 gehalten, so dass einerseits ein leichtes Einführen und Entnehmen der Behälter 2 und andererseits ein definierter Transport hinsichtlich der Geschwindigkeit und des Abstands der Behälter 2 untereinander sichergestellt werden kann. Die endlose Transportkette 5 wird dabei im Kreis gefahren.

Die Transporteinrichtung 4 ist nicht steril, so dass der aseptische Bereich der Füll- und Siegelzone 3b nur bis zur Transporteinrichtung 4 reicht, was aber für das sterile Abfüllen eines Lebensmittels in der Füll- und Siegelzone 3b ausreicht. Um eine Kontamination der Behälter 2 durch die Transporteinrichtung 4 zu vermeiden, wird in der Aseptikkammer 3 vorzugsweise eine Strömung von Sterilluft 7a von oben nach unten aufrechterhalten. Dazu sind entsprechende Sterilluftanschlüsse 8 entlang der Aseptikkammer 3 zur Zuführung von Sterilluft 7a vorgesehen. Infolge der Sterilluftströmung können keine Mikroorganismen von der Transporteinrichtung 4 nach oben wandern und sich am oberen Ende der Behälter 2 absetzen.

Die Sterilisationszone 3a und die Füll- und Siegelzone 3b sind bei der dargestellten Vorrichtung 1 durch einen Vorhang aus Sterilluft getrennt, die oben eingeblasen wird und im Wesentlichen laminar nach unten strömt. Alternativ oder zusätzlich zum Vorhang wäre auch eine Schleuse oder eine Engstelle denkbar, die gerade eben die Behälter in die Füll- und Siegelzone 3b durchlässt, die Atmosphäre aus der Sterilisationszone 3a jedoch wenigstens tendenziell zurückhält.

Nach dem Eintritt in die Sterilisationszone 3a werden die Behälter 2 durch eine Vorwärmeinrichtung 9 nacheinander durch Anblasen mit heißer Sterilluft 7a vorgewärmt. In einer nächsten Station werden die Behälter 2 mittels einer Sterilisiereinrichtung 10, die als Dosiereinrichtung ausgebildet sein oder eine Dosiereinrichtung umfassen kann, mit einer Mischung aus Wasserdampf, Wasserstoffperoxid und, vorzugsweise gefilterter Luft 7 beaufschlagt, um die Behälter 2 zu sterilisieren. Dazu wird bei der dargestellten und insoweit bevorzugten Vorrichtung 1 eine wässrige Wasserstoffperoxidlösung 11 mit einer Konzentration zwischen 30 Gew.-% und 40 Gew.-%, insbesondere von etwa 35 Gew.-%, Wasserstoffperoxid in einem Verdampfer 12 an einer elektrisch beheizten Oberfläche verdampft. Die Temperatur des Dampfes beträgt bei der dargestellten und insoweit bevorzugten Vorrichtung 1 zwischen 250°C und 300°C, etwa 270°C. Der Dampf wird zusammen mit der gefilterten Luft 7 aus einer Düse geblasen, um das Wasserstoffperoxid gleichmäßig über die zu sterilisierende Oberfläche der Behälter 2 zu leiten. Die gefilterte Luft 7 erhöht dabei das Gesamtvolumen des Sterilisiergases, das bei der dargestellten und insoweit bevorzugten Vorrichtung 1 eine Konzentration zwischen 2 Vol.-% und 10 Vol.-%, insbesondere 2,5 Vol.-% und 8 Vol.-%, beträgt. So kann eine Sterilisation der Behälter 2 bei geringem Einsatz von Wasserstoffperoxid erreicht werden. Das Wasserstoffperoxid reagiert an der Oberfläche des Behälters 2 bei Temperaturen zwischen 150°C und 270°C, insbesondere etwa 170°C bis 220°C, mit den dort vorhandenen Mikroorganismen und tötet diese dabei ab.

Nach der Sterilisation der Behälter 2 werden diese durch Beaufschlagen mit Sterilluft 7a über eine Trocknungseinrichtung 13 getrocknet, so dass das Wasserstoffperoxid und kondensiertes Wasser entfernt werden, bevor die Behälter 2 anschließend mit einer Fülleinrichtung 14 mit einem Lebensmittel, bei der dargestellten und insoweit bevorzugten Vorrichtung 1 einem Getränk, befüllt werden. Die befüllten Behälter 2 werden sodann verschlossen. Bei der dargestellten und insoweit bevorzugten Vorrichtung 1 erfolgt dies mit einer Verschließeinrichtung 15 durch Falten des oberen Bereichs der Packung und Siegeln des entsprechenden Bereichs, wobei einander berührende Packungsabschnitte miteinander verschweißt werden. Die verschlossenen Behälter 2 werden dann mittels der Transporteinrichtung 4 aus der Aseptikkammer 3 transportiert. Anschließend können die Behälter 2 aus den Zellen 6 der Transporteinrichtung 4 nacheinander entnommen werden.

Am unteren Ende der Sterilisationszone 3a, unterhalb der Transporteinrichtung 4 wird die Mischung aus Sterilluft, Wasserdampf und dampfförmigem Wasserstoffperoxid über einen Absaugkasten 16 abgezogen. Der Absaugkasten 16 erstreckt sich bei der dargestellten und insoweit bevorzugten Vorrichtung 1 nicht bis unter die Füll- und Siegelzone 3b. Es wird also nicht ebenfalls die Sterilluft aus der Füll- und Siegelzone 3b abgezogen, wodurch der über den Absaugkasten abgezogene Volumenstrom ansteigen und die Wasserstoffperoxidkonzentration infolge der entsprechenden Verdünnung absinken würde. Das Gasgemisch kann beispielsweise eine Temperatur von zwischen 50°C bis 80°C, insbesondere zwischen 60°C und 70°C aufweisen. Das abgezogene Gasgemisch wird sodann einem Kondensator 17 zugeführt, in dem das Wasser und das Wasserstoffperoxid teilweise kondensiert werden. Bei dem Kondensator kann es sich um einen Rohrbündelwärmetauscher mit mehreren Rohrregistern handeln. Bevorzugt ist es dabei, wenn die einzelnen Rohrregister jeweils im Kreuzstrom betrieben und untereinander seriell sowie im Gegenstrom zu dem zu kondensierenden Gasgemisch mit Kühlmittel durchströmt werden. Das zu kondensierende Gasgemisch wird dabei zickzackförmig von unten nach oben durch die einzelnen Rohrregister geführt. Dabei wird die Zusammensetzung des Gasgemischs über die der Sterilisationszone 3a zugeführten Gasströme und/oder die wenigstens eine Temperatur des Kondensators 17 so gewählt, dass die Konzentration des Wasserstoffperoxids im Kondensat 18 etwa zwischen 30 Gew.-% und 35 Gew.-% beträgt. Grundsätzlich können aber auch andere Konzentration wie beispielsweise wenigstens 15 Gew.-%, wenigstes20 Gew.-% oder wenigstens 25 Gew.-% sowie beispielsweise höchstens 70 Gew.-%, höchstens 50 Gew.-% oder höchstens 40 Gew.-% zweckmäßig sein.

Das Kondensat 18 wird anschließend einer Konditionierungseinrichtung 19 zugeführt, in der das Kondensat 18 in einer Filtereinrichtung 20 mit einem Filter aus gesintertem Polyethylen von Staub und/oder anderen Partikel aus der Sterilisationszone 3 befreit wird. Zur Reinigung des Filters kann dieser periodisch rückgespült werden.

Das so gereinigte Kondensat 18 wird zur Einstellung einer gewünschten Wasserstoffperoxidkonzentration einer Einstelleinrichtung 21 zugeführt, die bei der dargestellten und insoweit bevorzugten Vorrichtung 1 eine Verdünnungseinrichtung 22 und eine Konzentrierungseinrichtung 23 umfasst. Die Verdünnungseinrichtung 22 weist eine Zuführung 32 von, vorzugsweise demineralisiertem, Wasser in einen Vorlagebehälter 24 auf, in dem das Kondensat 18 aus dem Kondensator 17 durch Zugabe von Wasser verdünnt werden kann. Die Wasserstoffperoxidkonzentration wird dabei vorzugsweise auf einen Wert zwischen 30 Gew.-% und 40 Gew.-%, insbesondere etwa 35 Gew.-%, eingestellt. Die Konzentrierungseinrichtung 23 umfasst wenigstens eine Absorptionseinheit 25 umfassend Molekularsiebe zur Absorption von Wasser, das in einem separaten Schritt wieder aus den Molekularsieben ausgetrieben wird. Die aufkonzentrierte Wasserstoffperoxidlösung kann in den Vorlagebehälter 24 zurückgeleitet werden. Die Konditionierung der Wasserstoffperoxidlösung erfolgt diskontinuierlich und batchweise.

Aus dem Vorlagebehälter 24 wird die Wasserstoffperoxidlösung mit der eingestellten Konzentration zu einem Zwischenspeicher 27 geleitet, in den zum Ausgleich des verbrauchten und des in der den Kondensator 17 verlassenden Gasphase 25 enthaltenen Wasserstoffperoxids weitere Wasserstoffperoxidlösung durch eine Versorgungseinrichtung 28 nachgeführt werden. Dies erfolgt vorzugsweise über eine Wasserstoffperoxidlösung vorbestimmter Konzentration, die der Konzentration entsprechen kann, die im Vorlagebehälter 24 eingestellt wird.

Zudem können bei der dargestellten und insoweit bevorzugten Vorrichtung 1 mit dem Wasserstoffperoxid in Kontakt kommende Flächen der Vorrichtung 1 aus Stahl der Werkstoffnummer 1.4404 oder 1.4571 (jeweils V4A), aus Polyethylen (PE), aus Polypropylen (PP) oder aus Glas bestehen. Alternativ können die Kontaktflächen passiviert und/oder beheizt sein. Im Übrigen kann die Temperatur der Wasserstoffperoxidlösung zwischen dem Kondensator 17 und dem Verdampfer 12 aus einem Temperurniveau von kleiner 50°C gehalten werden, um ein Verdampfen von Wasserstoffperoxid zu vermindern. Alternativ oder zusätzlich können der Wasserstoffperoxidlösung organische und/oder anorganische Stabilisatoren zugegeben werden, die mit verdampft werden und/oder den Verdampfer 12 als Aerosole verlassen. Zur Förderung des Kondensats 18 vom Kondensator 17 zum Zwischenspeicher 27 und weiter zum Verdampfer 12 sind bei der dargestellten und insoweit bevorzugten Vorrichtung 1 Pumpen 29 vorgesehen. Diese Pumpen 29 können eine Zuführeinheit bilden oder ein Teil davon sein. Alternativ oder zusätzlich kann auch die Konditionierungseinrichtung 19 ganz oder teilweise Teil der Zuführeinheit sein. Gleiches gilt für entsprechende Rohrleitungen oder dergleichen, die zuvor nicht im Einzelnen erwähnt sind, der Fig. 1 aber ohne weiteres entnommen werden können. Zudem ist zum Abziehen von Wasserdampf, dampfförmigem Wasserstoffperoxid und Sterilluft aus der Sterilisationszone 3a bzw. dem Absaugkasten 16 eine Abzugseinrichtung 30 vorgesehen. Zum Betrieb der Konzentrierungseinrichtung 23 ist eine weitere Pumpe 31 vorgesehen.

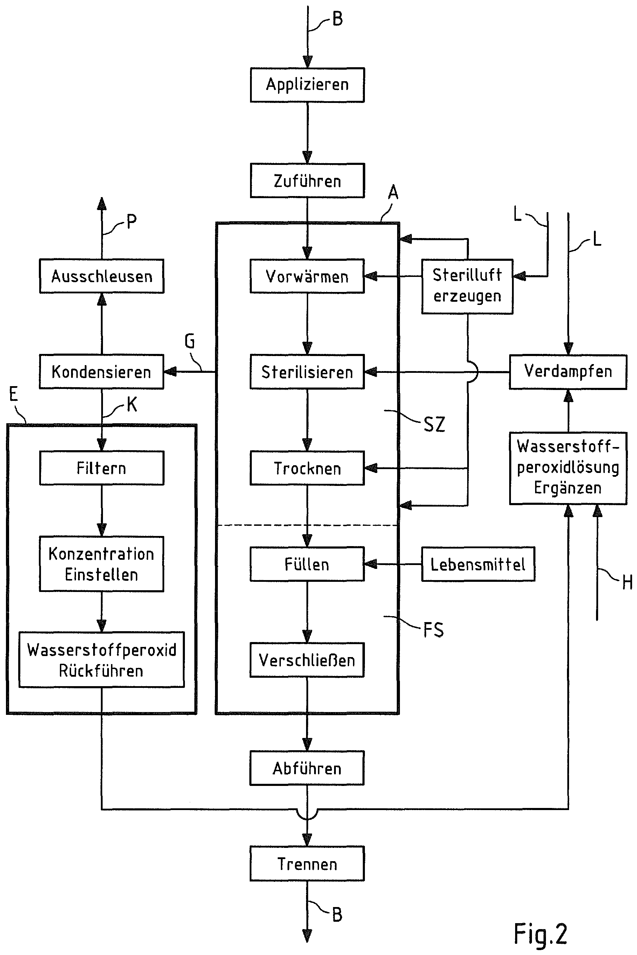

Das Verfahren zum Betrieb der zuvor beschriebenen Vorrichtung 1 ist in der Fig. 2 schematisch als Blockfließbild dargestellt. Dabei sind die in der Aseptikkammer oder in direkter Verbindung mit der Aseptikkammer erfolgenden Verfahrensschritte in einem die Aseptikkammer repräsentierenden Rahmen A angeordnet. Die Bearbeitungsschritte bezogen auf einen Behälter B sind vertikal untereinander angeordnet. Zunächst erfolgt die Applikation des Behälters B auf die Transporteinheit, die den Transport des Behälters B durch die Aseptikkammer A übernimmt. Sodann erfolgt die Zuführung des Behälters B in die Sterilisationszone SZ. In der Sterilisationszone SZ erfolgt die Vorwärmung des Behälters B mit heißer Sterilluft, die Sterilluft wird zunächst aus grob gereinigter Luft L durch filtrieren derselben hergestellt. Die Sterilluft wird auch an weiteren Stellen in die Aseptikkammer A eingeblasen.

Nach der Vorwärmung des Behälters B erfolgt die Sterilisation des vorgewärmten Behälters B mit einer Mischung aus grob gereinigter Luft, Wasserdampf und dampfförmigem Wasserstoffperoxid. Der sterilisierte Behälter B wird in einem weiteren Schritt in die Füll- und Siegelzone FS überführt und dort mit einem Lebensmittel aus einer Vorlage befüllt. Anschließend wird der Behälter B verschlossen und in einer Siegelstation durch Ultraschallschweißen versiegelt. Der so verschlossene Behälter wird mit Hilfe der Transporteinrichtung aus der Aseptikkammer A abgeführt. Danach erfolgt eine Trennung des fertigen Behälters B von der Transporteinrichtung.

Aus der Sterilisationszone SZ wird das enthaltene Gasgemisch G aus Wasserdampf, dampfförmigem Wasserstoffperoxid und Sterilluft abgezogen und einem Kondensator zugeführt. Den Kondensator verlassen das Kondensat K und eine Gasphase P, die bei der dargestellten und insoweit bevorzugten Vorrichtung als Abgas abgegeben wird. Die Gasphase P enthält neben der aus der Sterilisationszone SZ abgezogenen Luft noch Rest von Wasserdampf und Wasserstoffperoxid. Das Kondensat K wird in eine durch einen Rahmen symbolisierte Konditioniereinrichtung E überführt, in der eine Filtereinrichtung vorgesehen ist, die das Kondensat K filtert, um Feststoffe abzutrennen. Anschließend gelangt das gereinigte Kondensat in einen Vorlagebehälter zur Konditionierung des gereinigten Kondensats, insbesondere zur Einstellung der Wasserstoffperoxidkonzentration des Kondensats. Über eine Verdünnungseinrichtung kann die Konzentration des Wasserstoffperoxids im Kondensat mittels Zugabe von, insbesondere demineralisiertem Wasser herabgesetzt werden. Alternativ oder zusätzlich wird die Wasserstoffkonzentration des Kondensats angehoben, indem das Wasser des Kondensats teilweise abgeschieden wird, und zwar vorzugsweise gerade soweit, dass die gewünschte Wasserstoffperoxidkonzentration bereitgestellt werden kann. Die entsprechend konditionierte Wasserstoffperoxidlösung wird dann rückgeführt und in einen Zwischenspeicher abgegeben, aus dem die Wasserstoffperoxidlösung kontinuierlich dem Verdampfer zugeführt werden kann. Verbrauchtes Wasserstoffperoxid kann durch Zugabe einer frischen Wasserstoffperoxidlösung H geeigneter Konzentration an Wasserstoffperoxid in den Zwischenspeicher ersetzt werden. Die Konzentration der frischen Wasserstoffperoxidlösung entspricht dabei in etwa der Konzentration des konditionierten und dem Zwischenspeicher zugeführten Kondensats. Das Kondensat wird zum Sterilisieren weiterer Behälter erneut dem Verdampfer zugeführt.

## Patentansprüche

1. Verfahren zur Sterilisation von Behältern (2) zur Aufnahme von, insbesondere fließfähigen, Lebensmitteln, in einer Fülleinrichtung (14),
bei dem eine Wasserstoffperoxidlösung (11) in einem Verdampfer (12) verdampft wird,
bei dem in einer Sterilisationszone (3a) wenigstens ein Behälter (2) mit dem dampfförmigen Wasserstoffperoxid beaufschlagt wird,
bei dem der unverbrauchte Teil des dampfförmigen Wasserstoffperoxids wenigstens teilweise aus der Sterilisationszone (3a) abgezogen wird und
bei dem das abgezogene dampfförmige Wasserstoffperoxid in einem Kondensator (17) wenigstens teilweise kondensiert und das kondensierte Wasserstoffperoxid erneut dem Verdampfer (12) zugeführt wird.

2. Verfahren nach Anspruch 1,
bei dem die Sterilisationszone (3a), insbesondere zur Vorwärmung und/oder zur Trocknung des wenigstens einen Behälters, Sterilluft (7a) zugeführt wird und bei dem die Sterilluft (7a) wenigstens teilweise mit dem dampfförmigen Wasserstoffperoxids aus der Sterilisationszone (3a) abgezogen und dem Kondensator (17) zugeführt wird.

3. Verfahren nach Anspruch 1 oder 2,
bei dem eine wässrige Wasserstoffperoxidlösung (11) im Verdampfer (12) verdampft wird,
bei dem der unverbrauchte Teil des dampfförmigen Wasserstoffperoxids und der Wasserdampf jeweils wenigstens teilweise aus der Sterilisationszone (3a) abgezogen und in einem Kondensator (17) wenigstens teilweise kondensiert werden.

4. Verfahren nach einem der Ansprüche 1 bis 3,
bei dem das Wasserstoffperoxid bodenseitig, insbesondere unter einer Transporteinrichtung (4) zum Transport des wenigstens einen Behälters (2) durch die Vorrichtung (1) abgezogen wird.

5. Verfahren nach einem der Ansprüche 1 bis 4,
bei dem der Kondensator (17) derart betrieben wird, dass das Kondensat (18) eine höhere oder niedrigere Wasserstoffperoxidkonzentration als die Wasserstoffperoxidlösung (11) vor dem Verdampfen aufweist und bei dem, vorzugsweise, die Wasserstoffperoxidkonzentration wenigstens 10 Gew.-%, wenigstens 15 Gew.-%, wenigstens 20 Gew.-% oder wenigstens 25 Gew.-% und/oder höchstens 40 Gew.-%, höchstens 50 Gew.-% oder höchstens 70 Gew.-%beträgt.

6. Verfahren nach einem der Ansprüche 1 bis 5,
bei dem das im Kondensator (17) anfallende Kondensat (18) auf eine vorgegebene Wasserstoffperoxidkonzentration, vorzugsweise zwischen 25 Gew.-% und 50 Gew.-%, weiter vorzugsweise zwischen 30 Gew.-% und 40 Gew.-%, insbesondere 33 Gew.-% und 37 Gew.-%, eingestellt wird und, vorzugsweise, dass die Wasserstoffperoxidkonzentration des Konzentrats durch Verdünnen, insbesondere mit Wasser, und/oder durch Aufkonzentrieren, insbesondere durch ein Molekularsieb, eingestellt wird.

7. Verfahren nach einem der Ansprüche 1 bis 6,
bei dem das im Kondensator (17) anfallende Kondensat (18), insbesondere vor dem Einstellen, zur Abscheidung von Fremdstoffen gefiltert und/oder zum Ionentausch einem Ionentauscher, insbesondere Kationentauscher zugeführt wird.

8. Verfahren nach einem der Ansprüche 1 bis 7,
bei dem der wenigstens eine Behälter (2) in der Sterilisationszone (3a) vor dem Beaufschlagen mit dampfförmigem Wasserstoffperoxid mit heißer Sterilluft (7) vorgewärmt, nach dem Beaufschlagen mit dem dampfförmigen Wasserstoffperoxid mit Sterilluft (7) getrocknet, nach dem Beaufschlagen mit dem dampfförmigen Wasserstoffperoxid mit einem, insbesondere fließfähigen Produkt, gefüllt und/oder nach dem Befüllen verschlossen wird.

9. Verfahren nach einem der Ansprüche 1 bis 8,
bei dem mehrere Behälter (2) hintereinander mit Hilfe einer, vorzugsweise Zellen zur Aufnahme einzelner Behälter (2) aufweisenden, Transporteinrichtung (4), insbesondere Transportkette (5), durch die Sterilisationszone (3a), insbesondere die Aseptikkammer (3), transportiert werden.

10. Verfahren nach einem der Ansprüche 1 bis 9,
bei dem als Behälter (2) eine, vorzugsweise oben offene, Packung, insbesondere Kartonverbundpackung verwendet wird.

11. Vorrichtung zum Befüllen von sterilisierten Behältern (2) mit, insbesondere fließfähigen, Lebensmitteln, vorzugsweise zur Durchführung des Verfahrens nach einem der Ansprüche 1 bis 10, mit einer Sterilisationszone (3a) zur Aufnahme wenigstens eines Behälters (2), einem Verdampfer (12) zum Verdampfen einer Wasserstoffperoxidlösung (11), einer Sterilisiereinrichtung (10) zum Beaufschlagen des wenigstens einen Behälters (2) mit dampfförmigem Wasserstoffperoxid in der Sterilisationszone (3a) und einer Abzugseinrichtung (30) um wenigstens teilweisen Abziehen des unverbrauchten Teils des dampfförmigen Wasserstoffperoxids aus der Sterilisationszone (3a),
**dadurch gekennzeichnet, dass** ein Kondensator (17) zum wenigstens teilweisen Kondensieren des abgezogenen dampfförmigen Wasserstoffperoxids und einer Zuführeinheit zum Zuführen des kondensierten Wasserstoffperoxids zum Verdampfer (12) vorgesehen sind.

12. Vorrichtung nach Anspruch 11,
**dadurch gekennzeichnet, dass** eine Filtereinrichtung (19) zum Entfernen von Fremdstoffen aus dem Kondensat (18) des Kondensators (17) vorgesehen ist, eine Verdünnungseinrichtung (22) zum Verdünnen der Konzentration des kondensierten Wasserstoffperoxids auf eine vorbestimmte Konzentration, eine Konzentrierungseinrichtung (23) zum Anheben der Konzentration des kondensierten Wasserstoffperoxids und/oder eine Versorgungseinrichtung (28) zum Ersetzen von nicht kondensiertem Wasserstoffperoxid vorgesehen ist.

13. Vorrichtung nach Anspruch 11 oder 12,
**dadurch gekennzeichnet, dass** eine Vorwärmeinrichtung (9) zum Vorwärmen des wenigstens einen Behälters (2) mit heißer Sterilluft (7a) in der Sterilisationszone (3a), eine Trocknungseinrichtung (13) zur Trocknung des sterilisierten Behälters (2) mit Sterilluft (7a) in der Sterilisationszone (3a), eine Fülleinrichtung (14) zum Füllen des sterilisierten Behälters (2) mit Lebensmittel im Füll- und Siegelzone (3b) und/oder eine Verschließeinrichtung (15) zum Verschließen des gefüllten Behälters (2) im Füll- und Siegelzone (3b) vorgesehen ist.

14. Vorrichtung nach einem der Ansprüche 11 bis 13,
**dadurch gekennzeichnet, dass** eine vorzugsweise Zellen (6) zur Aufnahme einzelner Behälter (2) aufweisende, Transporteinrichtung (4), insbesondere Transportkette (5), zum Transport von mehreren Behältern (2) hintereinander durch die Sterilisationszone (3a) und/oder die Füll- und Siegelzone (3b), insbesondere die Aseptikkammer (3), vorgesehen ist.

15. Vorrichtung nach einem der Ansprüche 11 bis 14,
**dadurch gekennzeichnet, dass** wenigstens eine Steuereinrichtung zum Steuern und/oder Regeln des Verfahrens nach einem der Ansprüche 1 bis 10 vorgesehen ist.

## Claims

1. Method for sterilising containers (2) for receiving, in particular free-flowing, foods in a filling device (14),
wherein a hydrogen peroxide solution (11) is evaporated in an evaporator (12),
wherein, in a sterilisation zone (3a), at least one container (2) is exposed to the vaporous hydrogen peroxide,
wherein the unused part of the vaporous hydrogen peroxide is extracted at least in part from the sterilisation zone (3a), and
wherein the extracted vaporous hydrogen peroxide is condensed at least in part in a condenser (17), and the condensed hydrogen peroxide is supplied to the evaporator (12) again.

2. Method according to Claim 1,
wherein sterile air (7a) is supplied to the sterilisation zone (3a), in particular to preheat and/or dry the at least one container, and
wherein the sterile air (7a) is extracted at least in part together with the vaporous hydrogen peroxide from the sterilisation zone (3a) and supplied to the condenser (17).

3. Method according to either Claim 1 or 2,
wherein an aqueous hydrogen peroxide solution (11) is evaporated in the evaporator (12),
wherein the unused part of the vaporous hydrogen peroxide and the water vapour are each extracted at least in part from the sterilisation zone (3a) and condensed at least in part in a condenser (17).

4. Method according to any one of Claims 1 to 3,
wherein the hydrogen peroxide is extracted from the bottom, in particular underneath a conveyor apparatus (4) for conveying the at least one container (2) through the device (1).

5. Method according to any one of Claims 1 to 4,
wherein the condenser (17) is operated in such a way that the condensate (18) has a higher or lower hydrogen peroxide concentration than the hydrogen peroxide solution (11) before the evaporation, and wherein, preferably, the hydrogen peroxide concentration is at least 10 wt.%, at least 15 wt.%, at least 20 wt.% or at least 25 wt.% and/or at most 40 wt.%, at most 50 wt.%, or at most 70 wt.%.

6. Method according to any one of Claims 1 to 5,
wherein the condensate (18) accumulating in the condenser (17) is adjusted to a preset hydrogen peroxide concentration, preferably between 25 wt.% and 50 wt.%, more preferably between 30 wt.% and 40 wt.%, in particular 33 wt.% and 37 wt.%, and, preferably, wherein the hydrogen peroxide concentration of the concentrate is adjusted by dilution, in particular with water, and/or by concentration, in particular by a molecular sieve.

7. Method according to any one of Claims 1 to 6,
wherein the condensate (18) accumulating in the condenser (17), in particular before the adjustment, is filtered to separate impurities and/or is supplied to an ion exchanger, in particular a cation exchanger, for ion exchange.

8. Method according to any one of Claims 1 to 7,
wherein the at least one container (2), in the sterilisation zone (3a), is preheated with hot sterile air (7) before being exposed to vaporous hydrogen peroxide, dried with sterile air (7) after being exposed to the vaporous hydrogen peroxide, filled, with an in particular free-flowing product, after being exposed to the vaporous hydrogen peroxide and/or is sealed after being filled.

9. Method according to any one of Claims 1 to 8,
wherein a plurality of containers (2) are transported one after the other through the sterilisation zone (3a), in particular the aseptic chamber (3), by means of a conveyor apparatus (4), in particular a conveyor chain (5), preferably comprising cells for receiving individual containers (2).

10. Method according to any one of Claims 1 to 9,
wherein a packaging, in particular cardboard composite packaging, which is preferably open at the top, is used as a container (2).

11. Device for filling sterilised containers (2) with, in particular free-flowing, foods, preferably to carry out the method according to any one of Claims 1 to 10, comprising a sterilisation zone (3a) for receiving at least one container (2), an evaporator (12) for evaporating a hydrogen peroxide solution (11), a sterilising device (10) for exposing the at least one container (2) to vaporous hydrogen peroxide in the sterilisation zone (3a), and an extraction apparatus (30) to extract the unused part of the vaporous hydrogen peroxide from the sterilisation zone (3a) at least in part,
**characterised in that**
a condenser (17) to condense the extracted vaporous hydrogen peroxide at least in part, and a supply unit to supply the condensed hydrogen peroxide to the evaporator (12) are provided.

12. Device according to Claim 11,
**characterised in that**
a filter apparatus (19) is provided to remove impurities from the condensate (18) of the condenser (17), a dilution apparatus (22) is provided to dilute the concentration of the condensed hydrogen peroxide to a predetermined concentration, a concentration apparatus (23) is provided to raise the concentration of the condensed hydrogen peroxide and/or a supply apparatus (28) is provided to replace non-condensed hydrogen peroxide.

13. Device according to either Claim 11 or 12,
**characterised in that**
a preheating apparatus (9) is provided to preheat the at least one container (2) with hot sterile air (7a) in the sterilisation zone (3a), a drying apparatus (13) is provided to dry the sterilised container (2) with sterile air (7a) in the sterilisation zone (3a), a filling apparatus (14) is provided to fill the sterilised container (2) with food in the filling and sealing zone (3b) and/or a sealing apparatus (15) is provided to seal the filled container (2) in the filling and sealing zone (3b).

14. Device according to any of Claims 11 to 13,
**characterised in that**
a conveyor apparatus (4), in particular a conveyor chain (5), preferably comprising cells (6) for receiving individual containers (2), is provided to convey a plurality of containers (2) one after the other through the sterilisation zone (3a) and/or the filling and sealing zone (3b), in particular the aseptic chamber (3).

15. Device according to any of Claims 11 to 14,
**characterised in that**
at least one control unit is provided to control, in an open and/or closed loop manner, the method according to any of Claims 1 to 10.

## Revendications

1. Procédé de stérilisation de récipients (2) destinés à recevoir, en particulier des produits alimentaires liquides, dans un dispositif de remplissage (14),
dans lequel une solution de peroxyde d'hydrogène (11) évapore dans un évaporateur (12),
dans lequel au moins un récipient (2) est introduit avec le peroxyde d'hydrogène à l'état de vapeur dans une zone de stérilisation (3a) dans lequel la partie inutilisée du peroxyde d'hydrogène à l'état de vapeur est retirée au moins en partie de la zone de stérilisation (3a) et dans lequel le peroxyde d'hydrogène à l'état de vapeur retiré et condense au moins en partie dans un condensateur (17) et le peroxyde d'hydrogène condensé est de nouveau alimenté dans l'évaporateur (12).

2. Procédé selon la revendication 1,
dans lequel de l'air stérile (7a) est alimenté dans la zone de stérilisation (3a), en particulier pour le préchauffage et/ou le séchage d'au moins au récipient et dans lequel l'air stérile (7a) est retiré au moins en partie avec le peroxyde d'hydrogène à l'état de vapeur de la zone de stérilisation (3a) et alimenté dans le condensateur (17).

3. Procédé selon la revendication 1 ou 2,
dans lequel une solution aqueuse de peroxyde d'hydrogène (11) est évapore dans l'évaporateur (12)
dans lequel la partie inutilisée du peroxyde d'hydrogène à l'état de vapeur et la vapeur d'eau sont retirées au moins en partie de la zone de stérilisation (3a) et condensent au moins en partie dans un condensateur (17).

4. Procédé selon l'une des revendications 1 à 3,
dans lequel le peroxyde d'hydrogène au fond, en particulier sous un dispositif de transport (4) pour le transport d'au moins un récipient (2) à travers du dispositif (1) est retiré.

5. Procédé selon l'une des revendications 1 à 4,
dans lequel le condensateur (17) est commandé de manière à ce que le condensat (18) présente avant l'évaporation une concentration de peroxyde d'hydrogène supérieure ou inférieure à la solution de peroxyde d'hydrogène (11) et dans lequel, de préférence, la concentration de peroxyde d'hydrogène s'élève à au moins 10 % en poids, au moins 15 % en poids, au moins 20 % en poids ou au moins 25 % en poids et/ou au plus 40 % en poids, au plus 50 % en poids ou au plus 70 % en poids.

6. Procédé selon l'une des revendications 1 à 5,
dans lequel le condensat (18) présent dans le condensateur (17) est dosé sur une concentration de peroxyde d'hydrogène prédéfinie, de préférence entre 25 % en poids et 50 % en poids, puis de préférence entre 30 % en poids et 40 % en poids, en particulier 33 % en poids et 37 % en poids et, de préférence, la concentration de peroxyde d'hydrogène du concentré est dosé par dilution, en particulier avec de l'eau, et/ou concentration, en particulier par un tamis moléculaire.

7. Procédé selon l'une des revendications 1 à 6,
dans lequel le condensat (18) présent dans le condensateur (17), en particulier avant le dosage, est filtré pour la séparation d'impuretés et/ou alimenté dans un échangeur d'ions, en particulier un échangeur de cations pour l'échange d'ions.

8. Procédé selon l'une des revendications 1 à 7,
dans lequel au moins un récipient (2) est préchauffé dans la zone de stérilisation (3a) avant son introduction avec du peroxyde d'hydrogène à l'état de vapeur avec de l'air stérile (7) chaud, séché après son introduction avec le peroxyde d'hydrogène à l'état de vapeur avec de l'air stérile (7), rempli après l'application avec du peroxyde d'hydrogène à l'état de vapeur avec un produit, en particulier liquide, et/ou fermé après le remplissage.

9. Procédé selon l'une des revendications 1 à 8,
dans lequel plusieurs récipients (2) sont transportés les uns après les autres à l'aide d'un dispositif de transport, présentant de préférence des cellules pour accueillir des récipients (2) individuels, en particulier une chaîne de transport (5), à travers la zone de stérilisation (3a), en particulier la chambre aseptique (3).

10. Procédé selon l'une des revendications 1 à 9,
dans lequel un emballage, de préférence ouvert en haut, en particulier un emballage en matériau composite de carton, est utilisé comme récipient (2).

11. Dispositif de remplissage de récipients stérilisés (2) avec des produits alimentaires, en particulier liquides, de préférence pour l'exécution du procédé selon l'une des revendications 1 à 10, avec une zone de stérilisation (3a) destiné à accueillir au moins un récipient (2), un évaporateur (12) pour l'évaporation d'une solution de peroxyde d'hydrogène (11), un dispositif de stérilisation (10) pour l'introduction d'au moins un récipient (2) avec du peroxyde d'hydrogène à l'état de vapeur dans la zone de stérilisation (3a) et un dispositif d'extraction(30) pour le retrait au moins partiel de la partie inutilisée du peroxyde d'hydrogène à l'état de vapeur de la zone de stérilisation (3a),
**caractérisé en ce que**
un condensateur (17) pour la condensation au moins partielle du peroxyde d'hydrogène à l'état de vapeur retiré et une unité d'alimentation pour alimenter le peroxyde d'hydrogène condensé dans l'évaporateur (12) sont prévus.

12. Dispositif selon la revendication 11,
**caractérisé en ce que**
un dispositif de filtrage (19) pour éliminer les impuretés du condensat (18) du condensateur (17), un dispositif de dilution (22) pour diluer la concentration de peroxyde d'hydrogène condensé à une concentration prédéfinie, un dispositif de concentration (23) pour augmenter la concentration de peroxyde d'hydrogène condensé et/ou un dispositif d'alimentation (28) pour le remplacement du peroxyde d'hydrogène non condensé sont prévus.

13. Dispositif selon la revendication 11 ou 12,
**caractérisé en ce que**
un dispositif de préchauffage (9) pour préchauffer au moins un récipient (2) avec de l'air stérile (7a) chaud dans la zone de stérilisation (3a), un dispositif de séchage (13) pour sécher le récipient (2) stérilisé avec de l'air stérile (7a) dans la zone de stérilisation (3a), un dispositif de remplissage (14) pour remplir le récipient (2) stérilisé avec des produits alimentaires dans la zone de remplissage et de scellage (3b) et/ou un dispositif de fermeture (15) pour fermer le récipient (2) rempli dans la zone de remplissage et de scellage (3b)sont prévus.

14. Dispositif selon l'une des revendications 11 à 13,
**caractérisé en ce que**
un dispositif de transport présentant de préférence des cellules (6) pour accueillir des récipients (2) individuels, en particulier une chaîne de transport (5), pour le transport de plusieurs récipients (2) les uns après les autres à travers la zone de stérilisation (3a) et/ou la zone de remplissage et de scellage (3b), en particulier la chambre aseptique (3) est prévu.

15. Dispositif selon l'une des revendications 11 à 14,
**caractérisé en ce que**
au moins un dispositif de commande pour commander et/ou contrôler le procédé selon l'une des revendications 1 à 10 est prévu.
